# EUROPEAN PATENT APPLICATION

(11) **EP 3 252 135 A1**
(43) Date of publication of application: **06.12.2017**
(21) Application number: 16743146.9
(22) Date of filing: 07.01.2016
(51) Int. Cl.: C12G 3/00, C12G 1/00, C12G 3/02, C12M 1/00, C12P 7/06

(54) **ALCOHOLIC BEVERAGE HAVING ANTIOXIDATIVE FUNCTION, METHOD FOR REMOVING ACETALDEHYDE FROM ALCOHOLIC BEVERAGE AT LOW TEMPERATURE, REDUCTIVE FERMENTATION METHOD, AND OXIDATIVE/REDUCTIVE BREWING METHOD AND APPARATUS THEREFOR**

(30) Priority: 26.01.2015 JP 2015025811
(71) Applicant: Johokagaku Kenkyusyo Co. Ltd., Kumamoto-shi, Kumamoto 861-0134 (JP)
(72) Inventor: KAMIMURA, Chikashi, Kumamoto-shi Kumamoto 861-0134 (JP); KAMIMURA, Takashi, Kumamoto-shi Kumamoto 861-0134 (JP)
(74) Representative: Gulde & Partner
(86) International application number: PCT/JP2016/051196
(87) International publication number: WO 2016/121542

(57) **Abstract**

An alcoholic beverage containing ultrafine bubbles of hydrogen with an antioxidative function is provided that is produced by a reductive fermentation technique of alcohol and contains only a small amount of acetaldehyde. A method and an apparatus of oxidative-reductive fermentation of varieties of alcoholic beverages are provided based on facilitation of proliferation of microorganisms under an oxidative condition and a reductive fermentation technique. The reductive fermentation technique suppresses oxidative degradation of generated pyruvic acid, activate alcohol dehydrogenase, and facilitates production of alcohol from acetaldehyde which is an intermediate product, and a gas exchanging function of the ultrafine bubbles of hydrogen decreases acetaldehyde that remains in a period of alcohol fermentation. The technique facilitates fermentation, increases alcohol yield, decreases acetaldehyde content, and enhances fresh savor, mellow taste and an antioxidative function of an alcoholic beverage.

## Description

### Technical field

The present invention relates to an alcoholic beverage with an antioxidative function, a low-temperature removing method of acetaldehyde of an alcoholic beverage, a reductive fermentation method and an oxidative-reductive brewing method, and an apparatus therefore.

### Prior art

In fermentation brewing technology, quality of water for use is the most important, so that breweries were built at locations where water quality is so good that iron content is strictly small and no manganese is contained. In hard water fermentation proceeds rapidly, while in soft water fermentation proceeds slowly.

Rice wine, wine, distilled spirit, whisky, brandy and beer are different from one another in base material, however, they are all produced by saccharifying the base material into glucose and fermenting the glucose with use of yeast fungi.

Fermentation of rice wine is performed using rice as a base material through saccharifying action of Aspergillus oryzae and alcohol fermentation by yeast fungi, and as for conditions of the fermentation, a method to facilitate saccharification by Aspergillus oryzae under an aerobic condition, and proliferation of fungi and creation and administration of a reductive condition accompanying respiration in alcohol fermentation and the like are important.

As to fermentation of wine and brandy, alcohol fermentation of glucose of grape berries is performed by yeast fungi, and proliferation of fungi and creation and administration of a reductive condition accompanying respiration in alcohol fermentation are important.

Fermentation of distilled spirit is performed using rice, barley or sweet potato as a base material through saccharification by Aspergillus oryzae and alcohol fermentation by yeast fungi, where proliferation of fungi and creation and administration of a reductive condition accompanying respiration in alcohol fermentation are important.

In fermentation of whisky, barley is germinated, resulting malts are dried, smoked and then pulverized, and alcohol fermentation of glucose of the malts is performed by yeast fungi. Production of malts and proliferation of fungi as well as creation and administration of a reductive condition accompanying respiration in alcohol fermentation are important.

Also in fermentation of beer, barley is germinated and alcohol fermentation of glucose of malts is performed by yeast fungi. Also in beer, proliferation of fungi and creation and administration of a reductive condition accompanying respiration in alcohol fermentation are important.

As noted above, the part of saccharification varies depending on the species of alcoholic beverages, however, alcohol fermentation is performed by yeast fungi for all species. In the parts of these saccharification and fermentation, activity of microorganisms and self-fermentation of base materials are utilized, so that setting of biochemical conditions greatly affects a rate of fermentation, yield of alcohol and the like.

That is, efforts concerning the setting of conditions are technical know-how influencing fermentation.

Since alcoholic beverages are highly dependent on preference, variation in delicate savor and taste is considered important, and since microorganisms are involved, the center of the technology includes experiences over long years and intuitions on various matters such as a quality of malted rice, temperature of a fermentation tank, stirring, and how to determine timing.

Accordingly, many of administrations of fermentation place emphasis on how to derive good natural conditions, and as for facilities, automation is adopted and thus modernized with respect to those parts concerning growth of microorganisms, such as aseptic conditions, antibacterial techniques, precise management of temperature, mechanization of stirring and other works, however, enhancement of activity of microorganisms through an oxidative processing with minute gas bubbles or an activation technique of alcohol fermentation by reductive processing has not been introduced.

Further, with respect to brewed alcoholic beverages, removal of harmful acetaldehyde generated as an intermediate product is not performed by any method other than a distillation method, and left to natural progress of fermentation.

Patent Document 1 discloses an invention of the present inventors, based on which "hydrogen water itself" was first granted a patent, and the title of the invention is "Reductive Hydrogen Water For Food And The Like And Production Method And Production Apparatus Thereof". The method includes a technique in which hydrogen gas is blown into water, and stirring is performed to generate reductive hydrogen water. The method can be used in a reductive process of the present invention. The invention is now a Japanese Patent with No. 2890342.

Patent Document 2 discloses an invention of the present inventors, based on which production of hydrogen water with a function by minute gas bubbles was first granted a patent, and the title of the invention is "Auto-Oxidative/Reductive Processing System By Colloid Solution Of Decompression/Compression System Of Hydrogen Gas And Oxygen Gas". The method includes a technique in which gas is added to water in a decompressed state and stirring is performed by a pump to cause cavitation, and minute gas bubbles are generated by compression. The method can be used in an oxidative/reductive process of the present invention. The invention is now a Japanese Patent with No. 3843361.

Patent Document 3 discloses an invention of the present inventors, and the title of the invention is "Production Apparatus Of Hydrogen Solution With Free Radical Scavenging Ability". The method includes a technique in which hydrogen gas is added to water by an ejector and stirring is performed by a stirring rotor having a magnetic field to cause magnetic field cavitation, and water containing microbubbles of hydrogen with an antioxidative property is produced, the invention being now a Japanese Patent with No. 4309465.

In the present invention, no magnetic field is used and a generation source of an antioxidative property is ultra minute bubbles or ultrafine bubbles, therefore, there is a difference in size of gas bubbles, and the mechanism of the antioxidative property is basically different from that of Patent Document 3.

Patent Document 4 discloses an invention of the present inventors, and the title of the invention is "Hydrogen Colloid And Hydrogen Radical Colloid Of Viscous Solution And Production Method And Production System Therefor". The method includes a technique in which hydrogen gas is added by an ejector within a narrow stirring chamber and highspeed stirring is performed by a stirring rotor having a magnetic field to cause magnetic field cavitation, and microbubbles are generated to provide an antioxidative function to a viscous solution.

Patent Document 5 discloses an invention of the present inventors, and the title of the invention is "Production Method Of Nanobubbles By Vacuum Cavitation And Production Apparatus Of Nanobubbles". The method includes a technique in which two pumps are used, minute bubbles are generated by a preliminary pump and an ejector, reduced pressure or vacuum is generated by a secondary pump having a suction-discharging force greater than the primary pump, causing the minute bubbles to expand into vacuum breakup to produce nanobubbles whose median size is 1 µm or less. The method can be used in an oxidative/reductive process of the present invention.

Patent Document 1: Japanese Unexamined Patent Application Publication No. H08-056632
Patent Document 2: Japanese Unexamined Patent Application Publication No. 2004-344859
Patent Document 3: PCT International Publication No. WO2007/116889
Patent Document 4: Japanese Unexamined Patent Application Publication No. 2008-279424
Patent Document 5: Japanese Patent Application No. 2014-183658

### Summary

### Problems to be solved by the invention

When an alcoholic beverage is drunk, alcohol changes into acetaldehyde in the liver, causing a hangover. Since an oxidative condition exists in the liver, the following reaction proceeds

C₂H₅OH (alcohol) + NAD⁺ → CH₃CHO (acetaldehyde) + NADH + H⁺

so that rapid increase of acetaldehyde damages the liver, resulting in a hangover. That is, in a society of any generation, excessive drinking of alcoholic beverages causes diabetes as a lifestyle disease, and finally causes alcoholic intoxication, which leads to a tragedy of a miserable last half of life, as is often seen.

In order to prevent such cases, since alcohol does not increase acetaldehyde rapidly under a reductive condition, also in the liver, if a reductive condition is provided temporarily when alcohol comes in, rapid increase of acetaldehyde is suppressed, and accumulation of acetaldehyde in the liver does not arise. It can be assumed that alcohol can be decomposed slowly thereafter. Therefore, it is believed to be important that alcoholic beverages are subjected to a reductive process in advance so as to alleviate an oxidative condition in a tissue from which alcohol is first fed to the liver when the alcoholic beverage is drunk. Accordingly, one of the problems to be solved is to utilize a reductive alcoholic beverage containing ultrafine bubbles of hydrogen with an antioxidative function.

In alcoholic beverages, acetaldehyde, which is harmful to a human body, remains. As to removal of such acetaldehyde, if a reductive condition during fermentation is perfect, acetaldehyde changes to alcohol efficiently, however, if the reductive condition is not perfect, a large amount of acetaldehyde remains.

To remove remaining acetaldehyde, generally, there is a technique of fractional distillation of alcohol and acetaldehyde giving a distilled alcoholic beverage. However, if fractional distillation is performed at elevated temperature, savor essentially accompanying an alcoholic beverage is also removed.

Therefore, it is conventional knowhow that for a brewed alcoholic beverage, contents of a fermentation tank is fermented under a natural condition and a most reductive condition as far as possible to produce an alcoholic beverage containing a small amount of acetaldehyde.

The present invention relates to a technique where alcohol fermentation is performed under a forced reductive condition by adding hydrogen gas, and further, to a technique where remaining acetaldehyde is volatilized by gas exchange at low temperature to produce an alcoholic beverage containing a small amount of acetaldehyde, which are further problems to be solved.

In alcohol fermentation, steps of saccharification, addition of fermentation starters, proliferation of yeast, and alcohol fermentation proceed in this order. At the stage of proliferation of yeast, oxygen respiration occurs so that an aerobic condition is suitable, and supply of oxygen by minute bubbles of air or oxygen is necessary for growth of yeast.

That is, the stages of saccharification, addition of fermentation starters and proliferation of yeast proceed under an oxidative condition, and the stage of alcohol fermentation proceeds under a reductive condition. What is especially important is a strength of low reduction potential during alcohol fermentation. The chemical reaction formula of alcohol fermentation is as shown in Chemical Formula 1.

Chemical Formula 1 C₆H₁₂O₆ → 2C₂H₅OH + 2CO₂ ↑

From one molecule of glucose as the base material, two molecules of ethyl alcohol and two molecules of carbon dioxide gas is generated by fermentation enzymes of yeast.

The precise degradation process of the fermentation of Chemical Formula 1 proceed in three steps.

In the first step, as shown in Chemical Formula 2, one molecule of glucose is degraded into two molecules of pyruvic acid by a plurality of enzymes in the glycolytic system.

In this reaction, net two molecules of ADP are converted to ATP and simultaneously two molecules of NAD⁺ are converted to NADH. This stage is in common with the glycolytic pathway and the oxygen respiration pathway of animals and plants.

Chemical Formula 2 C₆H₁₂O₆ (glucose) + 2ADP + 2H₃PO₄ + 2NAD⁺ → 2CH₃COCOOH (pyruvic acid) + 2ATP + 2NADH + 2H₂O + 2H⁺

From the second stage, reactions particular to alcohol fermentation proceed.

In the second step, as shown in Chemical Formula 3, anaerobic respiration proceeds in which one molecule of carbon monoxide is removed from one molecule of pyruvic acid derived from glucose and acetaldehyde is generated.

This reaction is catalyzed by pyruvate decarboxylase under a reductive condition, as shown in Chemical Formula 3. Under an oxidative condition, aerobic respiration proceeds so that pyruvic acid is oxidatively degraded into carbon dioxide gas and water.

Chemical Formula 3 CH₃COCOOH (pyruvic acid) → CH₃CHO (acetaldehyde) + CO₂ ↑

In the third stage, as shown in Chemical Formula 4, acetaldehyde is rapidly reduced to ethanol by an electron of reduced form NADH.

In this reaction, alcohol dehydrogenation catalyst (alcohol dehydrogenase: an enzyme inherently catalyzing the reaction of oxidizing alcohol into acetaldehyde) generates alcohol by catalyzing an alcohol generating reaction from acetaldehyde under a reductive condition.

Chemical Formula 4 CH₃CHO (acetaldehyde) + NADH + H⁺ → C₂H₅OH (alcohol) + NAD⁺

With varieties of yeast, alcohol fermentation proceeds only under an anaerobic condition, and in the case under an aerobic condition, a reaction take places where pyruvic acid is degraded completely into water and carbon dioxide, leading to decreased production alcohol.

That is, in a usual fermentation method, yield of alcohol is lowered due to aerobic respiration.

For the reasons described above, the reaction of alcohol generation proceeds fast under a reductive condition. As a result, fermentation under a reductive condition improves yield of alcohol, so that it is believed that a reductive processing technique is necessary.

Accordingly, establishing an oxidative/reductive brewing technique is another problem to be solved, and it is a further problem where in the fermentation steps of various alcoholic beverages and how an oxidative process and a reductive process are performed.

### Means to solve the problem

### Alcoholic Beverage Containing Ultrafine Bubbles Of Hydrogen With Antioxidative Function

This is a technique, related to both a brewed alcoholic beverage and a distilled alcoholic beverage, of providing, as a method of suppressing a hangover, reductive radicals with an antioxidative function to the alcoholic beverage by feeding ultra minute bubbles of hydrogen gas having a size of 1 µm or less to an alcohol storage tank.

An apparatus used is a jet flow circulation processing apparatus of ultrafine bubbles of hydrogen gas generated by resonance foaming and vacuum cavitation. Further, since surface tension becomes smaller as a result of size reduction of water clusters due to ultrafine bubbles of hydrogen, and irritative taste of alcohol is masked by ultrafine bubbles of hydrogen, taste of the alcoholic beverage turns to extremely mellow.

### Low-Temperature Removing Method Of Acetaldehyde

This is a technique, related to both a brewed alcoholic beverage and a distilled alcoholic beverage, of feeding ultra minute bubbles of reductive hydrogen gas, nitrogen gas, carbon dioxide gas or the like having a size of 1 µm or less to an alcohol fermentation tank or a storage tank so as to volatilize acetaldehyde by gas exchange.

An apparatus used is, similarly to that for an alcoholic beverage containing ultrafine bubbles of hydrogen with an antioxidative function, a jet flow circulation processing apparatus of ultrafine bubbles of hydrogen gas generated by resonance foaming and vacuum cavitation.

### Reductive Fermentation Method

This is a technique in which, in alcohol fermentation, at a stage to generation of alcohol after proliferation of yeast, with use of a piston pump and a processing apparatus for minute bubbles of hydrogen gas, forced reductive processing of an inside of a fermentation tank is performed rapidly so as to reduce acetaldehyde and facilitate generation of alcohol.

Important points of this technique are a judgement of timing for switching between an oxidative condition and a reductive condition, and a rapid switching of oxidative/reductive levels.

In the method, purged hydrogen gas is circulated in a fermentation tank as ultra minute bubbles (micro bubbles) using a piston pump and a processing apparatus for minute bubbles of hydrogen gas, which instantaneously brings the inside of the fermentation tank to a strongly reductive state, causing acetaldehyde dehydrogenase and alcohol dehydrogenase to work favorably in a direction toward production of alcohol, which results in such advantages that glucose is efficiently converted to alcohol and that acetaldehyde, which is an intermediate product, is simultaneously decreased.

An apparatus is composed of a piston pump, a supply apparatus of hydrogen, and an ejector for generating microbubbles of hydrogen, and a processing technique was built up using a system for circulating an alcoholic beverage between the system including these apparatuses and an alcohol storage tank.

### Oxidative-Reductive Brewing Method

### 1) Preparation Step Of Base Material

Since alcohol fermentation includes a steaming step, oxidation of ingredients of a base material has influence on savor ingredients, greatly affecting product quality.

In order to prevent this, prior to steaming, a base material is immersed in water having been processed with ultrafine bubbles of hydrogen to realize a reductive condition in tissues of the base material, so as to suppress oxidation of ingredients at the steaming step and brew clear and transparent savor.

### 2) Producing Step Of Fermentation Starters

In preparation of fermentation starters, since proliferation of yeast requires a large amount of oxygen, air is fed and circulated to a fermentation starter tank by a piston pump for feeding microbubbles of air or oxygen and an ejector for generating microbubbles in water, so that fermentation starters including healthy yeast in high concentration are prepared.

### 3) Brewing Step Of Fermentation Mash

In alcohol fermentation, at an early stage of adding fermentation starters, microbubbles of air is fed and circulated by a piston pump and an ejector for generating microbubbles of air in water, so that proliferation of yeast is facilitated. Note that if there is a possibility that pasteurization bacteria arise, the processing is omitted.

At a stage where yeast has sufficiently proliferated, reductive processing by hydrogen gas is performed rapidly by a piston pump, a supply apparatus of hydrogen and an ejector for generating microbubbles of hydrogen in water, causing a reductive fermentation to proceed.

### 4) Posterior Step To Wringing Of Fermentation Mash

At a stage after fermentation mash is wrung and an unpasteurized alcoholic beverage is produced, a storage tank of the unpasteurized alcoholic beverage and a processing apparatus with ultrafine bubbles of hydrogen are connected and circulation reduction processing is performed, inhibiting intrusion of pasteurization bacteria and acetic bacteria in the unpasteurized alcoholic beverage, preventing rancidity of the alcoholic beverage and increasing preservative quality as an unpasteurized alcoholic beverage, so that the alcoholic beverage is made to have an antioxidative function, and thus, an alcoholic beverage good for health is provided.

### Advantageous effect of the invention

Removal of acetaldehyde for both a brewed alcoholic beverage and a distilled alcoholic beverage decreases acetaldehyde of the alcoholic beverage, which is harmful to a human body, and protects the liver, and additionally, a removing action of acetaldehyde itself produces an alcoholic beverage containing ultrafine bubbles of hydrogen having reductive radicals, so that an antioxidative function is exerted in a biological body and generation of acetaldehyde in the biological body is suppressed along with drinking of an alcoholic beverage, resulting in an alcoholic beverage hardly leading to a hangover, surface tension of the alcoholic beverage is lowered and alcohol is masked by the bubbles of hydrogen and becomes mellow, and ingredients that have a low boiling point and an irritating smell is decreased, resulting in mild savor.

Further, the antioxidative function provides the alcoholic beverage with a function exceeding that of conventional alcoholic beverages so called "the best medicine", and enables prevention of diseases of adults due to a scavenging function against active oxygen and enjoying antiaging.

The oxidative-reductive brewing method provides, through immersion processing of a base material in reductive water, advantageous effects of prevention of browning and decrease of functional ingredients due to oxidation of ingredients, and prevention of odor of oxidized lipid, making savor of an alcoholic beverage refreshing.

Processing collected water for fermentation starters with oxidative water has an advantageous effect of facilitating proliferation of yeast leading to enhancement of a fermentation function by healthy microorganisms.

Strong reduction processing by processing with microbubbles of hydrogen in reductive fermentation has advantageous effects of directing enzymes of yeast toward alcohol fermentation, not only enhancing the function of alcohol fermentation and increasing yield of alcohol, but also preventing intrusion of other putrefactive bacteria, leading to inhibition of deterioration by spoiling bacteria and inhibition of rancidity of a product by acetic bacteria. As for a brewed alcoholic beverage, shipment even as an unpasteurized alcoholic beverage becomes possible, and maturation thereof is effected very much.

### Brief description of drawings

Fig. 1 shows a simplified oxidative-reductive processing apparatus in a small-scale brewing experiment.
Fig. 2 is a chart of a jet flow circulation processing apparatus of ultrafine bubbles of nitrogen gas, carbon dioxide gas or hydrogen gas for alcoholic beverages.
Fig. 3 is a chart of an oxidative/reductive fermentation processing apparatus of fermentation starters/mash by a piston pump and a spiral mixer.
Fig. 4 is an illustration of application of oxidative/reductive processing in an oxidative/reductive brewing step of producing rice wine.
Fig. 5 is an illustration of application of oxidative/reductive processing in an oxidative/reductive brewing step of producing wine.
Fig. 6 is an illustration of application of oxidative/reductive processing in an oxidative/reductive brewing step of producing distilled spirit.
Fig. 7 is an illustration of application of oxidative/reductive processing in an oxidative/reductive brewing step of producing whisky.
Fig. 8 is an illustration of application of oxidative/reductive processing in an oxidative/reductive brewing step of producing a distilled alcoholic beverage such as brandy.
Fig. 9 is an illustration of application of oxidative/reductive processing in an oxidative/reductive brewing step of producing beer.

### Description of reference numerals

1: maturing tank/storage wood tank for distilled alcoholic beverage
2: fermentation tank or maturation tank/storage wood tank for brewed alcoholic beverage
3: supply apparatus of gas
4: generation apparatus of ultrafine bubbles by resonance foaming and vacuum cavitation
5: reduction processing apparatus of fermentation starters/mash by piston pump, resonance ejector and resonance foaming apparatus
6: spiral mixer
7: suction circulation pipe
8: discharge circulation pipe
9: spouted alcoholic beverage after atomization processing of gas bubbles
10: fermentation mash being brewed, brewed alcoholic beverage being matured, or distilled alcoholic beverage being matured
11: insect-preventing/dust-preventing gas-discharging open-close apparatus for ventilation of fermentation tank, maturation tank, wood tank

### <Reference numerals of gas supply system>

12: gas bomb
13: main tap of gas
14: gas pressure gauge of gas bomb
15: gas pressure reducing valve
16: gas pressure reducing gauge
17: gas flowmeter
18: needle valve
19: gas purging apparatus
20: gas lead pipe

### <Reference numerals of vacuum cavitation apparatus, reduction processing apparatus of fermentation mash>

21: resonance ejector
22: resonance foaming apparatus
23: primary pump for resonance foaming
24: secondary pump for vacuum cavitation
25: piston pump for processing of fermentation mash
26: electric power source of apparatus

### Detailed description of preferred embodiments

### <Production Method Of Alcoholic Beverage Containing Ultrafine Bubbles Of Hydrogen With Acetaldehyde Removing Function And Antioxidative Function>

An alcoholic beverage with an antioxidative function is an alcoholic beverage produced in the following way: ultrafine bubbles of hydrogen gas are generated and injected forcibly into an alcoholic beverage so that a supersaturation state of minute bubbles of hydrogen gas is created in the alcoholic beverage, providing a function of reductive radicals.

It was found that gas exchange by processing with ultrafine bubbles replaces and volatilizes acetaldehyde, volatile ingredients with a very irritative odor, irritative savor ingredients and the like that are contained in alcohol and have a low boiling point, yielding fragrance with a less irritative odor even in a process period of 10 to 60 minutes.

It was found that, in processing with ultrafine bubbles of hydrogen, despite that an alcoholic hydroxyl group (-OH) usually gives a strong irritative taste to the tongue, such an irritative taste is masked by the ultrafine bubbles containing supersaturated hydrogen so that a mild taste without an irritative taste is yielded even in a process period of 10 to 60 minutes. Further, ingredients having a low boiling point and an irritative odor were decreased, to yield mild savor.

Moreover, it was found that while processing an alcoholic beverage with ultrafine bubbles of hydrogen changes the alcoholic beverage into a strongly reductive state of -600 mV or lower, minute gas bubbles of nanosize are brought into a supersaturation state of hydrogen, so that reactivity of the minute gas bubbles against substances is increased and ingredients of the alcoholic beverage acquire an antioxidative function, generating a scavenging ability against DPPH radicals.

As to a production method, in a case of a distilled alcoholic beverage, a storage tank 1 or storage wood tank 1 after distillation is used, and in a case of a brewed alcoholic beverage, a storage tank 1 or alcohol fermentation tank 2 is used, which is connected to circulation pipes 7, 8 and a processing apparatus 4 with ultrafine bubbles so that ultrafine bubbles of hydrogen are added and circulation processing is performed for 10 to 120 minutes or so, which brings the alcoholic beverage into a supersaturation state of hydrogen gas and the ultrafine bubbles of hydrogen generate reductive radicals having an antioxidative function.

By the processing, volatile savor such as acetaldehyde contained in alcohol are volatilized and removed from the alcoholic beverage through gas exchange. In order to further decrease acetaldehyde, which is a cause of a hangover, long term circulation processing of 60 minutes or more is further performed, which is continued until an aimed level is reached.

Thus, it was confirmed that by processing an alcoholic beverage with ultrafine bubbles of hydrogen gas, even in a short process period of 10 to 60 minutes, acetaldehyde is decreased due to gas exchange, an irritative odor in savor is also decreased making the savor refreshing, hydroxyl groups which are a cause of an irritative taste of alcohol are masked yielding a mellow taste, and scavenging ability against active oxygen is generated, which is useful for advancement of health, yielding an alcoholic beverage with an antioxidative function.

The gas used in removing acetaldehyde may be not only hydrogen gas but also nitrogen gas or carbon dioxide gas, which also exerts a gas exchanging function by the same processing and shows the same effect, however, generation of reductive radicals is effected only by ultrafine bubbles of hydrogen.

### <Production Apparatus Of Alcoholic Beverage Containing Ultrafine Bubbles Of Hydrogen With Acetaldehyde Removing Function And Antioxidative Function>

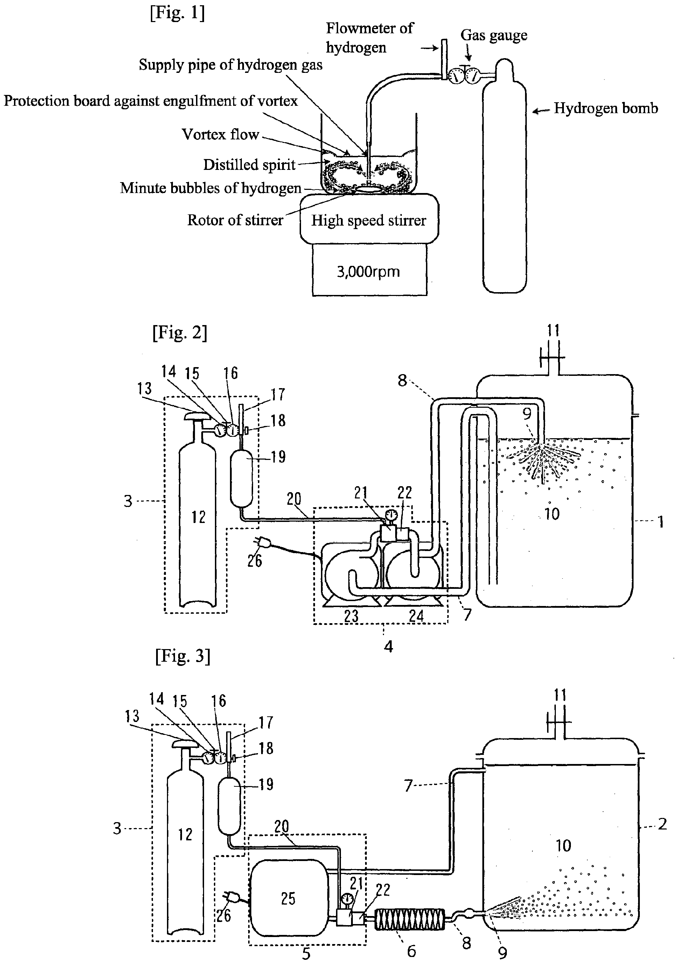

For processing with ultrafine bubbles in which reductive radicals are generated, a jet flow circulation processing apparatus of ultrafine bubbles for alcoholic beverages shown in Fig. 2 is used.

The principle is as follows: a maturation tank/ storage wood tank 1 of an alcoholic beverage, a primary pump 23 configured to suck fermentation mash and feed it to a processing apparatus, and a vacuum cavitation apparatus employing a resonance foaming apparatus 22 and a secondary pump 24 are connected to perform circulation.

A production apparatus of an alcoholic beverage containing ultrafine bubbles is a systematic apparatus in which a storage tank of an alcoholic beverage 1 and an apparatus for resonance foaming and vacuum cavitation are combined.

The apparatus sucks a solution out of the storage tank and the wood tank by the primary pump 23 to spout it into a resonance ejector 21, resonance foaming is caused in a mixed solution of gas and liquid by the resonance foaming apparatus 22, vacuum is formed by the secondary pump 24 having a strong suction power in uniform microbubbles generated to make the micro gas bubbles expand to tens of times, which are crushed by vacuum cavitation, and ultrafine bubbles of hydrogen gas are generated through crushing operation performed in twice.

As to the configuration, a storage tank 1 or a storage wood tank 1, a circulation pipe, a resonance foaming apparatus and a vacuum cavitation apparatus are connected into a system, to spout and circulate the ultrafine bubbles of hydrogen in a stored alcoholic beverage to fill the stored alcoholic beverage entirely with the ultrafine bubbles of hydrogen.

The functions are removal of acetaldehyde by gas exchange and alcohol fermentation; the latter generates two molecules of ethyl alcohol and two molecules of carbon dioxide gas from one molecule of glucose as the base material by means of fermentation enzymes of yeast, the precise degradation proceeding in three stages.
(1) A reaction to degrade one molecule of glucose into two molecules of pyruvic acid by a plurality of enzymes in the glycolytic system.
(2) A reaction to remove one molecule of carbon dioxide from pyruvic acid to generate acetaldehyde by acetaldehyde dehydrogenase.
(3) A reaction to generate alcohol from acetaldehyde by alcohol dehydrogenase.

In these reactions, acetaldehyde dehydrogenase generates acetaldehyde from pyruvic acid under a reductive condition. Alcohol dehydrogenase generates alcohol from acetaldehyde under a reductive condition.

That is, if a reductive condition is not set rapidly, a fairly large amount of alcohol is degraded into carbon dioxide gas and water, which causes a loss. Therefore, introduction of a strongly reductive condition facilitates the reaction of generating alcohol from acetaldehyde by means of the action of alcohol dehydrogenase, so that the function of generating alcohol is promoted.

Further, rapid setting of a reductive condition has a function to inhibit the activities of spoiling bacteria, preventing putrefaction during fermentation and adhesion of a bad smell.

Further, since proliferation of acetic bacteria in an unpasteurized alcoholic beverage of rice wine or wine is suppressed, a sour taste does not increase during storage, which enables shipment as the unpasteurized alcoholic beverage (prevention of rancidity of alcoholic beverages).

Therefore, in the present invention, at the stage where a sufficient number of yeast fungi is acquired, said reduction processing is performed, and by creating a reductive condition rapidly, a reductive fermentation is performed to facilitate generation of alcohol and increase yield of alcohol. An alcoholic beverage produced through reductive fermentation is an antioxidative reductive alcoholic beverage having a strong reductive action.

### <Processing Apparatus For Alcoholic Beverage Containing Ultrafine Bubbles Of Hydrogen>

Fig. 2 shows a jet flow circulation processing apparatus of ultrafine bubbles to generate reductive radicals.

In processing with ultrafine bubbles by this apparatus, homogeneous minute gas bubbles of nanosize can be generated, and it is found that minute gas bubbles generated in processing of an alcoholic beverage with ultrafine bubbles of hydrogen generate reductive radicals, providing a strong antioxidative function to the alcoholic beverage.

As to the storage tank 1, hydrogen gas supplied during processing and carbon dioxide gas due to fermentation pool at the upper portion of the tank, so that a gas-discharging open-close apparatus 11 configured to be insect-preventing/dust-preventing is arranged on top of the tank for gas ventilation.

### <Reductive Fermentation Technique>

For reduction processing in a fermentation tank, an oxidative-reductive brewing apparatus of fermentation mash in Fig. 3 is used. A piston pump 5, a spiral mixer 6 and a circulation pipe 7 are used for sucking out fermentation mash 9 under preparation in the fermentation tank 1, nitrogen gas, carbon dioxide gas or hydrogen gas from a supply apparatus 2 of purged gas of nitrogen gas, carbon dioxide gas or hydrogen gas is injected by a resonance ejector 21 connected to the piston pump 5 and the spiral mixer 6 to generate microbubbles of primary minute gas bubbles (containing mainly gas bubbles of 50 µm or less) of hydrogen gas, nitrogen gas or carbon dioxide gas.

Alcohol fermentation generates two molecules of ethyl alcohol and two molecules of carbon dioxide gas from one molecule of glucose as the base material by fermentation enzymes of yeast, the precise degradation proceeding in three stages.
(1) A reaction to degrade one molecule of glucose into two molecules of pyruvic acid by a plurality of enzymes in the glycolytic system.
(2) A reaction to remove one molecule of carbon dioxide from pyruvic acid to generate acetaldehyde by acetaldehyde dehydrogenase.
(3) A reaction to generate alcohol from acetaldehyde by alcohol dehydrogenase.

In these reactions, acetaldehyde dehydrogenase generates acetaldehyde from pyruvic acid under a reductive condition. Alcohol dehydrogenase generates alcohol from acetaldehyde under a reductive condition.

That is, if a reductive condition is not set rapidly, a fairly large amount of alcohol is degraded into carbon dioxide gas and water, which causes a loss. Further, introduction of a strongly reductive condition facilitates the reaction of generating alcohol from acetaldehyde by means of the action of alcohol dehydrogenase, so that the function of generating alcohol is promoted.

Moreover, rapid setting of a reductive condition has a function to inhibit the activities of spoiling bacteria, preventing putrefaction during fermentation and adhesion of a bad smell.

Furthermore, since proliferation of acetic bacteria in an unpasteurized alcoholic beverage of rice wine or wine is suppressed, a sour taste does not increase, which enables shipment as the unpasteurized alcoholic beverage.

Therefore, in the present invention, at the stage where a sufficient number of yeast fungi is acquired, said reduction processing is performed, and by creating a reductive condition rapidly, a reductive fermentation is performed to facilitate generation of alcohol and increase yield of alcohol. An alcoholic beverage produced through reductive fermentation is an antioxidative reductive alcoholic beverage having a strong reductive action.

### <Reductive Fermentation Apparatus>

The reductive fermentation apparatus of fermentation mash in Fig. 3 is composed of an alcohol fermentation tank 1, a supply apparatus 2 of purged gas of nitrogen gas, carbon dioxide gas or hydrogen gas, a reductive fermentation apparatus 5 of mash including a piston pump for producing minute gas bubbles of nitrogen gas, carbon dioxide gas or hydrogen gas and a spiral mixer, and circulation pipes 7, 8 which connect the fermentation tank 1, the piston pump 25, the resonance ejector 21, a resonance foaming apparatus 22, and the spiral mixer 6.

The supply apparatus 3 of purged hydrogen gas is composed of a gas bomb 12, a gas gauge 14, a gas purging apparatus 19, and a gas supply pipe 20, and the operation includes the followings.
(1) A gas main tap 13 of the gas bomb 12 is opened and a gas pressure of the gas in the bomb is confirmed by the gas pressure gauge 14.
(2) A gas pressure reducing valve 15 is opened to adjust the gas pressure to an aimed pressure while watching a gas pressure reducing gauge 16.
(3) A needle valve 18 is opened to adjust a gas flow to an aimed flow while watching a gas flow gauge 17.
(4) After operation, similarly to start of operation, the gas main tap 13 is closed, the gas pressure reducing valve 15 is closed, the needle valves 18 is closed, and it is conformed that output of gas is completely ceased, thus completing operation.

The reductive fermentation apparatus 5 of fermentation mash is composed of an electric power supply 26, the piston pump 25, the ejector 21, and the spiral mixer 6.

Operation is performed as follows: the electric power supply 26 is set to on to activate the piston pump 25, fermentation mash 10 under preparation is sucked out of the alcohol fermentation tank 1 by the circulation pipe 7, and hydrogen gas fed from the supply apparatus 3 of purged hydrogen gas is made into microbubble hydrogen mash by the ejector 21 and spouted 9 and added to the fermentation mash 10 under preparation by the pipe 8.

The microbubble hydrogen fermentation mash added to the alcoholic beverage 10 under preparation turns to strongly reductive due to circulation operation of the system, so that reductive fermentation proceeds when -400 mV to -600 mV is totally reached.

### <Oxidative/Reductive Brewing Technique Of Alcoholic Beverages>

### (1) Reductive Processing Of Base Material

As for rice wine and distilled spirit, grain such as rice, barley and buckwheat or sweet potato is steamed, and saccharification is performed by Aspergillus oryzae, which is followed by a brewing step.

Especially, contamination by nitrogen compounds and lipids of bran portions is unfavorable for rice, so that rice is subjected to polishing. For rice wine called "ginjo-shu", made from highly polished rice, 40 % of the base material is removed as bran. A main reason therefor is that ingredients are oxidized in the stage of steaming, and odor of oxidized lipid and smell of potato adversely affect the savor of the alcoholic beverage.

In the present invention, for the water in which a base material is immersed before steaming, water containing ultrafine bubbles of hydrogen processed by the "resonance foaming apparatus and vacuum cavitation apparatus" 4 in the jet flow circulation processing apparatus of ultrafine bubbles shown in Fig. 2 is used, so that even the central portion of a grain is placed under a reductive condition.

In the grain of which even the central portion has been placed under a reductive condition, oxidation hardly occurs during steaming so that occurrence of odor of oxidized lipid is prevented. As a result, rice becomes pure white and the alcoholic beverage produced therefrom has refreshing savor.

### (2) Facilitation Of Malt Making

The steamed base material is cooled to 33 to 36°C rapidly, spores of Aspergillus oryzae are mixed uniformly, so that saccharification and generation of citric acid are facilitated. The malt making has a great influence on fermentation of mash and requires supply of sufficient humidity and sufficient oxygen, and spray of ultrafine bubbles of oxygen by resonance foaming/vacuum cavitation is suitable for humidification.

### (3) Facilitation Processing Of Yeast Proliferation

Prior to alcohol fermentation, healthy growth of fermentation starters is necessary. The fermentation starters are for the purpose of growing yeast strong, so that for collected water for fermentation starters, water of ultrafine bubbles of air or oxygen by the "resonance foaming apparatus and vacuum cavitation apparatus" in the jet flow circulation processing apparatus of ultrafine bubbles shown in Fig. 2 is used, and thus strongly oxidative water is supplied.

Proliferation speed of yeast becomes twice or three times the speed in conventional natural proliferation.

For strengthening of yeast, an oxidative condition in a fermentation tank is necessary at initial fermentation. For oxidative processing of the fermentation tank, the oxidative/reductive fermentation apparatus 5 of fermentation mash by the piston pump and the spiral mixer shown in Fig. 3 is used, and fermentation mash 9 under preparation is sucked out of the fermentation tank 1 by the circulation pipe 8, and air or oxygen is mixed in.

Note that, if there is a possibility of occurrence of spoiling bacteria, the oxidative processing of the fermentation tank is canceled.

### (4) Facilitation Processing Of Reductive Fermentation

For reduction processing of the fermentation tank, the oxidative/reductive fermentation apparatus 5 of fermentation mash by the piston pump and the spiral mixer shown in Fig. 3 is used, and the fermentation mash 10 under preparation is sucked out of the fermentation tank 1 by the circulation pipe 8, and gas is mixed in.

For facilitation of yeast proliferation, air or oxygen gas from the supply apparatus 3 of air or oxygen is injected by the resonance ejector 21, and microbubbles are generated by the resonance foaming apparatus 22, which is spouted into the fermentation tank 1 as microbubbles of air or oxygen 9 by the circulation pipe 8, and thus, the microbubbles of air or oxygen is circulated to the fermentation tank 1 to facilitate proliferation of yeast. Note that, if there is a possibility of occurrence of spoiling bacteria, the oxidative processing of the fermentation tank is canceled.

In reductive fermentation, the same apparatus is used while changing species of the gas used. As for the gas for reductive fermentation, all of hydrogen gas, nitrogen gas and carbon dioxide gas have a fermentation facilitating function, however, hydrogen gas is the most excellent for establishing a reductive condition, and when hydrogen gas is used, acetaldehyde is changed rapidly to alcohol.

### <Oxidative-Reductive Brewing Apparatus By Facilitation Of Proliferation Of Microorganisms Under Oxidative Condition And Reductive Fermentation Technique>

Brewing of various alcoholic beverages such as rice wine, wine, distilled spirit, whisky and beer commonly converts sugar/starch of base materials to glucose and performs fermentation with use of yeast fungi.

Since proteins, lipids and other ingredients are contained in addition to sugar/starch in the base material, and these may cause accidents in the stages from preparation of the base material to saccharification and fermentation such as occurrence of abnormal odor, change in taste, and rancidity, therefore, administration techniques to prevent such accidents are very important.

Among them is an oxidative-reductive brewing method, in which an oxidative condition and a reductive condition are arranged appropriately in the steps to perform positively an oxidative processing and a reductive processing.
(1) Brewing techniques for rice wine, distilled spirit and beer include a step of heating and steaming a base material of fermentation. In the step of heating and steaming, lipids, proteins and other ingredients contained in the base material give rise to oxidative browning.
   In the oxidative browning, sweaty smell, odor of oxidized lipid, smell of potato and the like are generated and adversely affects savor of an undistilled alcoholic beverage, so that oxidation-preventing processing of the base material by forced reduction using the "resonance foaming apparatus and vacuum cavitation apparatus" in the jet flow circulation processing apparatus of ultrafine bubbles shown in Fig. 2 is necessary.
(2) In the stages of production of malt spores, malt making, production of fermentation starters, addition of yeast and the like, since facilitation of proliferation of microorganisms is necessary, an oxidative condition by stirring with air is required, so that mixing of fermentation mash or the like has been performed since old times. That is, oxidative facilitating processing is necessary for facilitating proliferation of microorganisms by forced oxidation using the "resonance foaming apparatus and vacuum cavitation apparatus" shown in Fig. 2.
(3) In the stage of alcohol fermentation, three steps of reactions are necessary: generation of pyruvic acid from alcohol by yeast in the course of glycolysis, generation of acetaldehyde through decarboxylation of pyruvic acid, and generation of alcohol through reduction of acetaldehyde. In particular, generation of alcohol through reduction of acetaldehyde does not proceed if a condition in the fermentation tank is not reductive. Thus, providing a reductive condition is necessary. In the brewing nowadays, only creation of a reductive condition that accompanies respiration of yeast fulfills the condition. With such passive reduction, carbon dioxide gas and water are generated, lowering yield of alcohol. Therefore, rapid and forced reduction processing is required using the oxidative/reductive fermentation brewing apparatus 5 of fermentation mash by the piston pump and the spiral mixer shown in Fig. 3.

In order to administrate oxidative-reductive processing at each stage of the processing steps, rapid oxidative processing and rapid reductive processing are necessary, and an apparatus is required for communicating and circulating between the production apparatus for ultrafine bubbles of air, oxygen and hydrogen and the fermentation tank.

Ultrafine bubbles has a function of dissolving oxidative gas and reductive gas in solutions very rapidly, and enables rapid and forced oxidative processing and reductive processing.

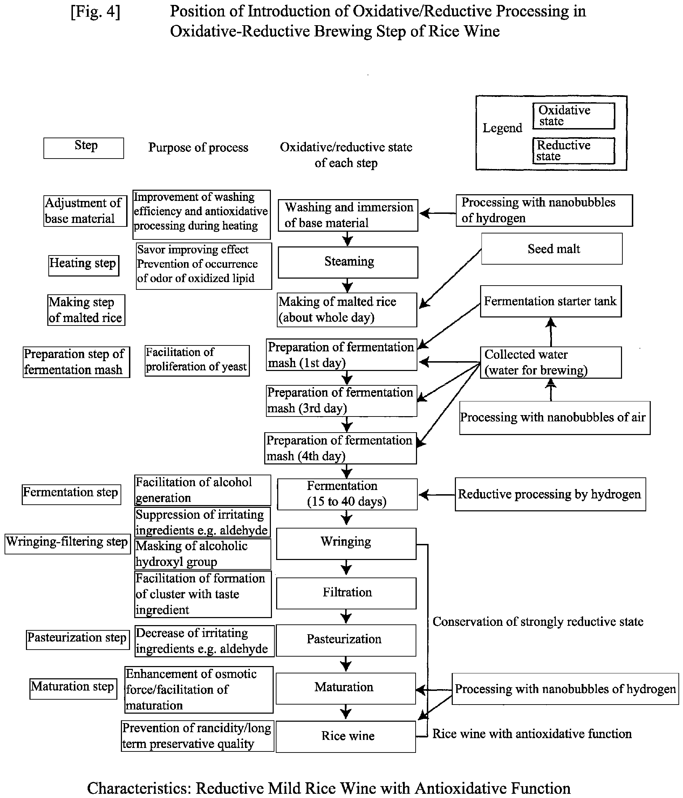

Figs. 4 to 9 show positions in steps for various alcoholic beverages where processing with ultrafine bubbles of air/hydrogen is applied, and positions are depicted where an oxidative state by ultrafine bubbles of air needed and a reductive state by ultrafine bubbles of hydrogen gas is needed.

In the figures, provision of an oxidative state by ultrafine bubbles of air is indicated by a reddish color, and provision of a reductive state by ultrafine bubbles of hydrogen gas is indicated by a dark blueish color.

The continuous oxidative/reductive processing apparatus of the jet flow circulation processing apparatus of ultrafine bubbles shown in Fig. 2 is composed of the alcohol storage tank 1, the supply apparatus 3 of purged hydrogen gas for producing ultra minute gas bubbles of air, oxygen and hydrogen gas, the production apparatus 4 of ultrafine bubbles, and the pipes 6, 7 to circulated the ultra minute gas bubbles.

Operation is performed as follows: an electric power switch is turned on so that collected water is sucked out of the alcohol storage tank 1 by the primary pump 23 or an alcoholic beverage 9 to be matured is sucked out by the pump of the production apparatus 4 of ultrafine bubbles through the pipe 7, and hydrogen, air or oxygen is supplied from the supply apparatus 3 of gas and ultrafine bubbles are generated by the production apparatus 4 of ultrafine bubbles.

As to a generation method of ultrafine bubbles, the valve 13 of the bomb of the supply apparatus 3 of gas is opened and a gas pressure of the gas in the bomb is confirmed by the gas pressure gauge 14. The gas pressure reducing valve 15 is opened to adjust the gas pressure to an aimed pressure while watching the gas pressure reducing gauge 16. The needle valve 18 is opened to adjust a gas flow to an aimed flow while watching the gas flow gauge 17. With the supplied gas, a mixed solution of gas and liquid is formed by the resonance ejector 21, and microbubbles are generated by the resonance foaming apparatus 22, causing white turbidity. In the generated water with microbubbles, vacuum is generated by a strong suction force of the secondary pump, and the gas bubbles expand and are subjected to vacuum cavitation caused by the secondary pump 24, turning into ultrafine bubbles or ultra minute gas bubbles. The ultrafine bubbles thus generated contains a large amount, namely 6 % or more, of gas, however, no white turbidity exists therein.

Fig. 3 shows an oxidative/reductive processing fermentation apparatus of alcoholic beverages by microbubbles of hydrogen, air or oxygen, as a processing apparatus by microbubbles.

Using these apparatuses, oxidative/reductive processing is performed rapidly and forcibly, depending on necessity of the steps of each alcoholic beverage.

A base material, fermentation mash or a fermented alcoholic beverage 9 is subjected to processing with microbubbles of a suitable gas needed at each step, homogenized by the spiral mixer 6, and circulated to the fermentation tank 1 through the pipe 7. Supplying process of gas is similar to that for the production apparatus 4 of ultrafine bubbles.

As to a direction of circulation, while observing a status of fermentation, the system is reassembled based on a judgement of a technical expert, and a circulation direction can also be selected such that a liquid at the upper portion of the fermentation tank is sucked out and spouted into the liquid at the lower portion of the fermentation tank.

In principle, circulating the liquid at the lower portion of the fermentation tank to the liquid at the upper portion of the fermentation tank is a method to eliminate influence of air on the liquid at the upper portion caused by the fact that the liquid surface comes in contact with air.

Sucking the liquid at the upper portion of the fermentation tank to circulate and spout to the liquid at the lower portion of the fermentation tank has a function of raising nanobubbles gently in the liquid, and thus, this circulation direction is believed to be advantageous in uniformizing oxidative-reductive conditions between the upper portion and the lower portion of the fermentation tank.

The processing apparatus with microbubbles in the present oxidative/reductive processing is applicable both in a system where it is arranged movably for each fermentation tank and in a system where it is arranged fixedly at each fermentation tank.

### <Oxidative-Reductive Brewing Method Of Rice Wine By Facilitation Of Proliferation Of Microorganisms Under Oxidative Condition And Reductive Fermentation Technique>

Detailed processes of producing steps in oxidative-reductive brewing method of rice wine is shown in Fig. 4.

In brewing rice wine, it is important in which steps the production apparatus processing with ultrafine bubbles of an oxidative condition that facilitates proliferation of microorganisms and the processing with nanobubbles of a reductive condition that facilitates alcohol fermentation are used.

Brewing of rice wine starts with polishing of a base material, and about 30 % is polished (rice-polishing rate 70 %) for common rice wine/average rice wine, and 40 % or more is polished for ginjo-shu. Polish of the base material directly affects the quality of ingredients contained.

As shown in Fig. 4, the steps proceed from washing and immersion, via steaming, making of malted rice, preparation of fermentation mash, fermentation, wringing, filtration, pasteurization, maturation, and filling, to shipping.

In general, for washing and immersion, famous water of brewery's pride is used but no special processing is performed. However, this is followed by the step of steaming, so that ingredients of rice are oxidized if unprocessed, and the savor is adversely affected by occurrence of odor of oxidized lipid or smell of potato. For this reason, polishing of the base material is required.

The washing, immersing and steaming processing of the base material in the present oxidative-reductive brewing method is performed as follows: the base material to be brewed is washed with reductive water provided by the jet flow circulation processing apparatus of ultra bubbles of hydrogen shown in Fig. 2 and the base material is immersed in reductive water, so that, through prevention of oxidation of functional ingredients of rice during steaming, protection and prevention of browning is achieved, and savor is improved.

In producing malt spores and making malt, proliferation of Aspergillus oryzae is facilitated by use of oxidative water provided by the production apparatus of ultrafine bubbles of air or oxygen (Fig. 2) for improvement of propagation of fungi.

In producing fermentation starters, proliferation of yeast fungi is facilitated by using oxidative water provided with ultrafine bubbles of air or oxygen (Fig. 2) as water for brewing in order to improve growth of yeast and to grow strong fungi.

At the initial stage of preparation of mash, it is important that yeast fungi grow strongly in the whole of the fermentation tank, and thus, proliferation of yeast fungi is facilitated by use of oxidative water provided with ultrafine bubbles of air or oxygen for promotion of respiration.

When alcohol fermentation is started, since generation of alcohol from acetaldehyde, which is an intermediate product, by alcohol dehydrogenase proceeds under a reductive condition, the fermentation tank is subjected to rapid and forced reduction by reductive processing with ultrafine bubbles of hydrogen (Fig. 3).

The rapid and forced reduction (Fig. 3) removes even acetaldehyde that remains due to incomplete fermentation by a gas exchanging function of ultrafine bubbles of hydrogen, so that acetaldehyde content decreases.

Rice wine produced is highly reductive so that proliferation of acetic bacteria is suppressed, and rancidity is never caused.

As for maturation of rice wine, the jet flow circulation processing apparatus with ultrafine bubbles shown in Fig. 2 is used.

Processing with ultrafine bubbles that generates reductive radicals provides DPPH radical-scavenging ability to the ingredients of rice wine, so that acetaldehyde content is decreased, characteristically yielding rice wine with an antioxidative function.

### <Oxidative-Reductive Brewing Method Of Wine By Facilitation Of Proliferation Of Microorganisms Under Oxidative Condition And Reductive Fermentation Technique>

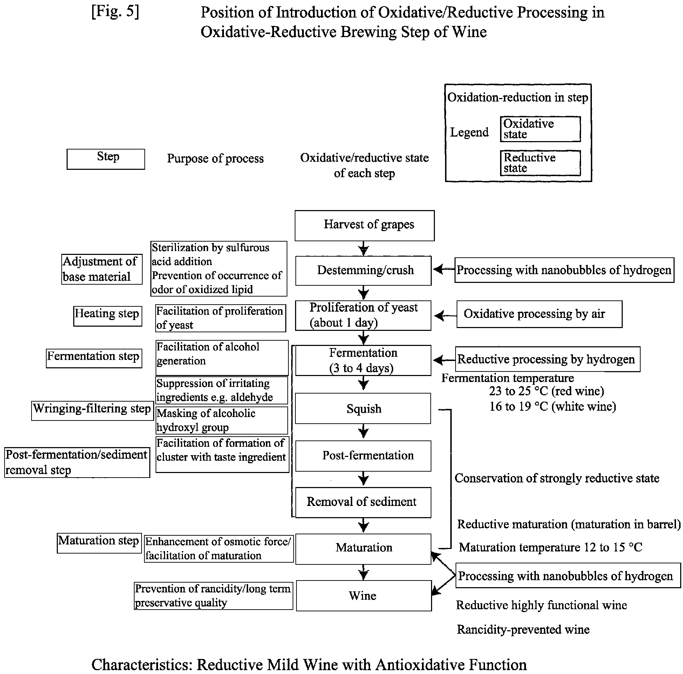

Detailed processes of producing steps in oxidative-reductive brewing method of wine is shown in Fig. 5.

In brewing wine, it is important in which steps the processing with ultrafine bubbles of an oxidative condition that facilitates proliferation of microorganisms and the processing by ultrafine bubbles of a reductive condition that facilitates alcohol fermentation are used.

As shown in Fig. 5, the steps proceed from harvest of grapes, via destemming, crush, addition of yeast, proliferation of yeast, fermentation, squish, post-fermentation, removal of sediment, and maturation to filling.

At the stages of destemming/crush of the base material, there is a possibility of oxidation of functional ingredients of grapes, occurrence of abnormal odor, or rancidity, which are prevented, however, by using reductive water provided with ultrafine bubbles of hydrogen (Fig. 2).

Since proliferation of fermentation starters proceeds faster under an oxidative condition, processing with ultrafine bubbles of air or oxygen is performed partially at the step of proliferation of yeast. However, in a case where suppression of acetic bacteria is necessary, it is safer to perform processing with ultrafine bubbles of hydrogen in all steps.

When alcohol fermentation is started, since alcohol dehydrogenase that converts acetaldehyde, which is an intermediate product, to alcohol requires a reductive condition, the fermentation tank is subjected to rapid and forced reduction by microbubbles of hydrogen, to facilitate generation of alcohol by reductive fermentation (Fig. 3).

Remaining acetaldehyde is also removed by the gas exchanging function of microbubbles of hydrogen so that acetaldehyde content is decreased.

Wine produced is highly reductive so that proliferation of acetic bacteria is suppressed, and rancidity is never caused.

As for maturation of wine, the processing apparatus with ultrafine bubbles that generates reductive radicals is used. The jet flow circulation processing apparatus with ultrafine bubbles shown in Fig. 2 provides DPPH radical-scavenging ability to alcohol in wine to protect ingredients with an antioxidative function contained in wine, such as anthocyanin.

In reductive fermentation of wine, acetaldehyde content is decreased, maturation of wine is accelerated by the jet flow processing with ultrafine bubbles applied to the undistilled alcoholic beverage, and mellowness in taste and mild savor are brewed, so that wine with an antioxidative function is characteristically yielded.

### <Oxidative-Reductive Brewing Method Of Distilled Spirit By Facilitation Of Proliferation Of Microorganisms Under Oxidative Condition And Reductive Fermentation Technique>

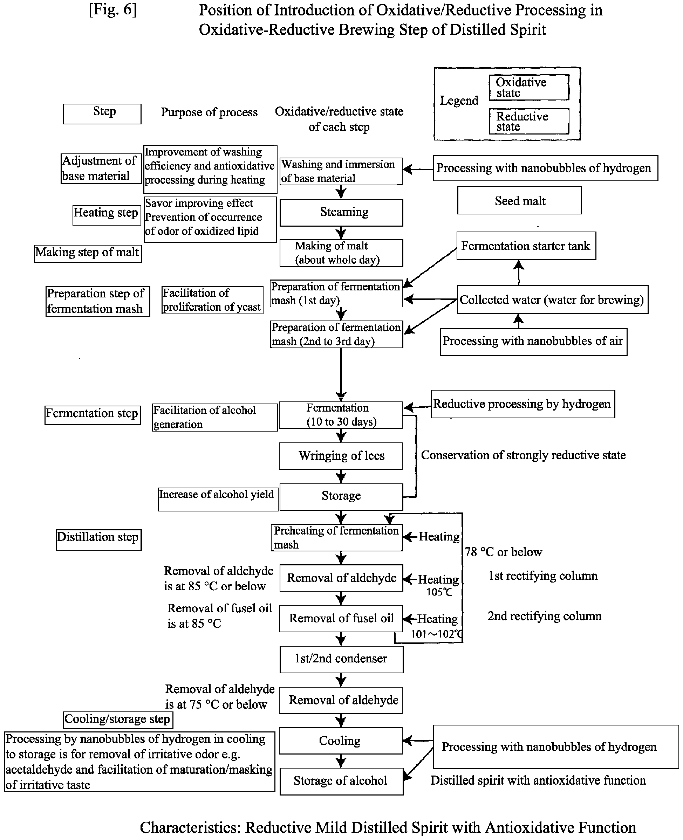

Detailed processes of producing steps in oxidative-reductive brewing method of distilled spirit is shown in Fig. 6.

Distilled spirit uses different base materials such as rice, barley or sweet potato, so that fermentation steps thereof are also different from those in brewing of rice wine. However, both are the same in basic parts such as saccharification of starch and alcohol fermentation of glucose generated through saccharification, and thus, some parts are common between brewing distilled spirit and brewing rice wine.

As shown in Fig. 6, the following steps are included: washing and immersion of a base material (for sweet potato, washing and crushing of the base material), steaming, making of malt, preparation of fermentation mash, fermentation, wringing of lees, storage, preheating of fermentation mash, distillation removal of aldehyde, distillation removal of fusel oil, condensation, distillation removal of aldehyde, cooling, storage of alcohol, and maturation.

Washing/immersion of the base material is performed by the jet flow circulation processing apparatus (Fig. 2) with ultrafine bubbles of hydrogen to improve savor through protection by prevention of oxidation of ingredients and inhibition of browning during steaming.

In producing malt spores, proliferation of Aspergillus oryzae is facilitated by use of oxidative water provided with microbubbles of air or oxygen (Fig. 2) so as to produce strong malt spores.

In production of fermentation starters, oxidative water containing ultrafine bubbles of air or oxygen (Fig. 2) is used as water for brewing, so that proliferation of yeast fungi is facilitated leading to growth of strong fermentation starters.

At an initial stage of preparation of mash, oxidative water containing ultrafine bubbles of air or oxygen (Fig. 2) is used, so that proliferation of yeast fungi is facilitated.

In alcohol fermentation, the fermentation tank is subjected to rapid reduction by reductive water provided by the oxidative/reductive fermentation processing apparatus (Fig. 3) for fermentation starters/mash using the piston pump and the spiral mixer, to facilitate generation of alcohol from acetaldehyde, which is an intermediate product, through reductive fermentation by alcohol dehydrogenase, shortening a preparation period and a fermentation period and increasing yield of alcohol.

Reductive processing removes remaining acetaldehyde by the gas exchanging function of fine bubbles of hydrogen at low to ordinary temperature, yielding an undistilled alcoholic beverage with small acetaldehyde content.

The undistilled alcoholic beverage produced is subjected to distillation. After distillation, reductive processing is performed in a wood tank by the jet flow circulation processing apparatus with ultrafine bubbles of hydrogen (Fig. 2) so as to achieve facilitation of maturation as well as enhancement of the function of ingredients with an antioxidative function such as phenolic compounds of the wood tank by hydrogen radicals, yielding a product as an alcoholic beverage with reductive radicals.

### <Oxidative-Reductive Brewing Method Of Whisky By Facilitation Of Proliferation Of Microorganisms Under Oxidative Condition And Reductive Fermentation Technique>

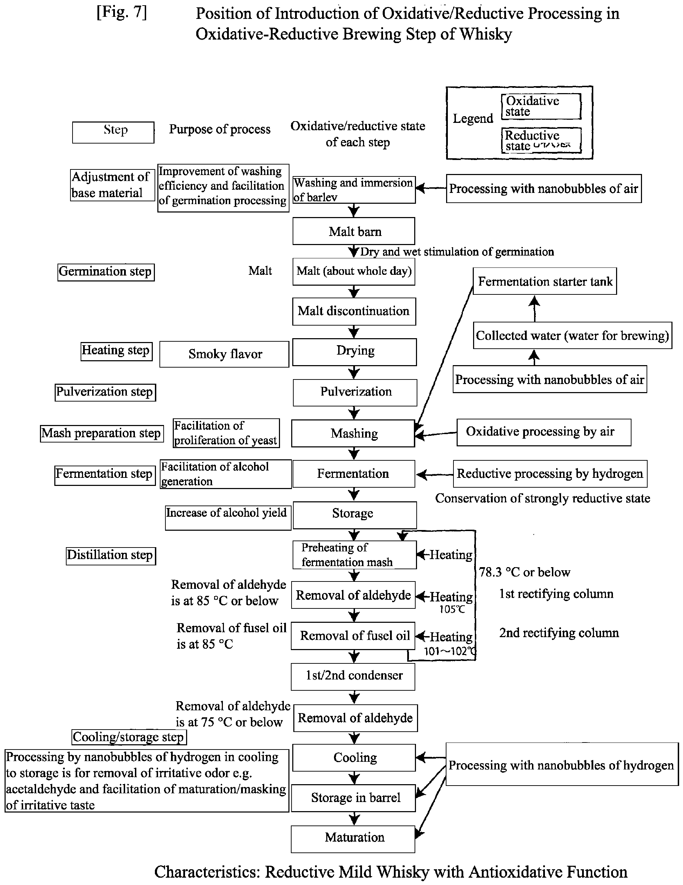

Detailed processes of producing steps in oxidative-reductive brewing method of whisky is shown in Fig. 7.

As shown in Fig. 7, brewing steps of whisky proceed in the following order: washing of barley/malt barn, production of malt, germination arresting, drying, pulverization, mashing, fermentation, storage, preheating, distillation removal of aldehyde, distillation removal of fusel oil, condensation, distillation removal of aldehyde, cooling, storage in barrel, and maturation.

In brewing of whisky, growth of malt is important.

In washing/immersion of barley, the base material, oxidative water provided by the jet flow circulation apparatus of ultrafine bubbles of air (Fig. 2) is used to grow malt under an oxidative condition.

In mashing, the jet flow circulation apparatus of ultrafine bubbles of air (Fig. 2) is used to facilitate proliferation of yeast fungi.

In alcohol fermentation, the oxidative/reductive fermentation processing apparatus of fermentation starters/mash by the piston pump and the spiral mixer (Fig. 3) is used to achieve saturation with microbubbles of hydrogen, the fermentation tank is subjected to rapid and forced reduction, so as to facilitate generation of alcohol from acetaldehyde, which is an intermediate product, through the reductive fermentation by alcohol dehydrogenase.

The effect of oxidative processing with ultrafine bubbles of air or oxygen (Fig. 2) at the initial stage of mashing facilitates proliferation of yeast, and thus, shortens the mashing period.

The processing with microbubbles of hydrogen (Fig. 3) in alcohol fermentation enhances yield of alcohol and removes remaining acetaldehyde by the gas exchanging function of microbubbles of hydrogen, yielding an undistilled alcoholic beverage with small acetaldehyde content.

The undistilled alcoholic beverage produced is subjected to distillation. After distillation, reductive processing is performed in a wood tank by the jet flow circulation processing apparatus with ultrafine bubbles of hydrogen (Fig. 2) to facilitate maturation and strengthen the function of antioxidative ingredients such as phenolic compounds of the wood tank by hydrogen radicals, giving rise to a product as an alcoholic beverage with reductive radicals.

### <Oxidative-Reductive Brewing Method Of Distilled Alcoholic beverage Such As Brandy and Method of Providing Antioxidative Function>

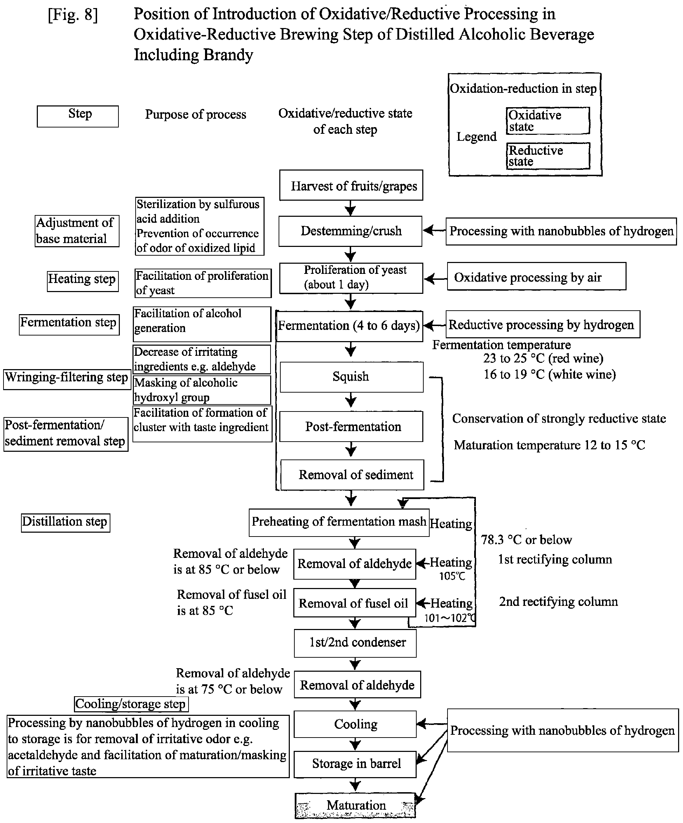

Processing method at producing steps in oxidative-reductive brewing method of distilled alcoholic beverages such as brandy is shown in Fig. 8.

Brandy is a distilled alcoholic beverage that is made from wine or fruit liquor as a base material. Therefore, steps for producing fruit liquor are similar to those in the production method of wine. As to the steps after distillation, steps common in distilled alcoholic beverages will be described.

As shown in Fig. 8, the steps proceed in the following order: harvest of grape (fruit), destemming, crush, addition of yeast, proliferation of yeast, fermentation, squish, post-fermentation, removal of sediment, storage, preheating, distillation removal of aldehyde, distillation removal of fusel oil, condensation, distillation removal of aldehyde, cooling, storage in barrel, and maturation.

At the stages of destemming/crush of the base material, there is a possibility of occurrence of abnormal odor or rancidity due to oxidation of functional ingredients of grapes, which are prevented, however, by using reductive water provided with nanobubbles of hydrogen.

Since proliferation of fermentation starters proceeds faster under an oxidative condition, at the stage of producing fermentation starters, oxidative processing is applied to collected water using the jet flow circulation processing apparatus with ultrafine bubbles of air (Fig. 2). However, in a case where suppression of spoiling bacteria/acetic bacteria is necessary, it is believed to be safer to perform processing with microbubbles of hydrogen (Fig. 3) in all steps.

When alcohol fermentation is started, since alcohol dehydrogenase that converts acetaldehyde, which is an intermediate product, to alcohol requires a reductive condition, fermentation tank is subjected to rapid and forced reduction by the oxidative/reductive fermentation processing apparatus (Fig. 3) by the piston pump and the spiral mixer for hydrogen for fermentation starters/mash, to facilitate generation of alcohol through reductive fermentation. Fruit liquor produced is subjected to distillation. After distillation, maturation is facilitated in a wood tank by use of the jet flow circulation processing apparatus with ultrafine bubbles of hydrogen (Fig. 2) and the function of antioxidative ingredients such as phenolic compounds of the wood tank is strengthened by hydrogen radicals, yielding a product as an alcoholic beverage with reductive radicals.

In producing distilled alcoholic beverages with reductive radicals having an antioxidative function, reductive fermentation is always performed before distillation, and an antioxidative function is provided by reductive processing with ultrafine bubbles of hydrogen after distillation.

### <Oxidative-Reductive Brewing Method Of Beer By Facilitation Of Proliferation Of Microorganisms Under Oxidative Condition And Reductive Fermentation Technique>

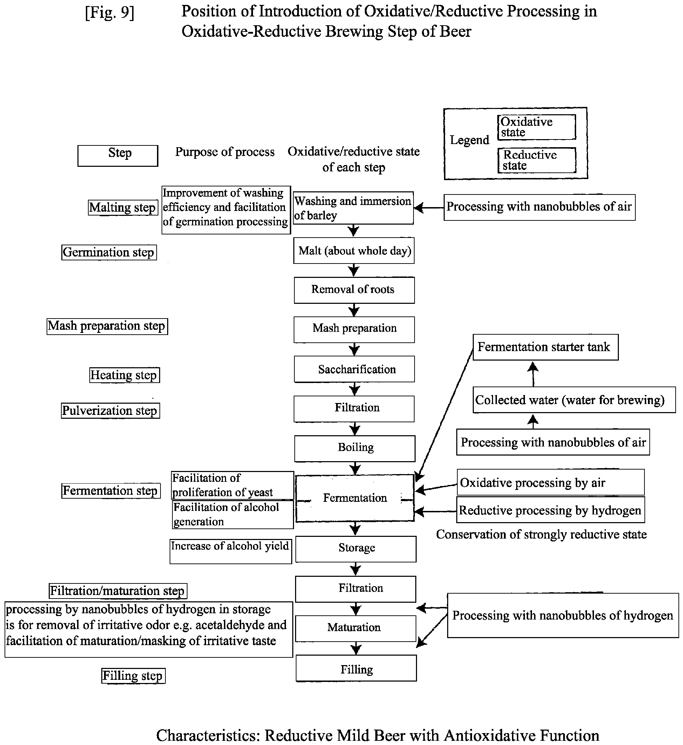

Detailed processes of producing steps in oxidative-reductive brewing method of beer is shown in Fig. 9.

As shown in Fig. 9, brewing steps of beer proceed in the following order: washing/immersion of barley, production of malt, removal of roots, preparation of malt, saccharification, filtration, boiling (hops are added), fermentation, storage, filtration, maturation, and filling.

In brewing of beer, oxidative water provided with ultrafine bubbles of air or oxygen (Fig. 2) is used for growth of malt and washing/immersion of the base material.

An oxidative condition is preferred for growth of malt, so that sprouting is forced under an oxidative condition, then preparation is performed, saccharification is facilitated, and filtration is performed. After filtration, hops and the like are added to wort and reductive processing is performed with microbubbles of hydrogen (Fig. 3).

Reductive processing (Fig. 3) suppresses occurrence of odor of oxidation caused by steaming ingredients of wort, hops and the like. After boiling, alcohol fermentation is performed.

In alcohol fermentation, at the initial stage, the jet flow circulation processing apparatus with ultrafine bubbles of air (Fig. 2) is used to facilitate proliferation of yeast fungi, and at the late stage, the jet flow circulation processing apparatus with ultrafine bubbles of hydrogen (Fig. 2) is used to perform rapid and forced reduction of the fermentation tank.

Reductive processing (Fig. 3) of the fermentation tank facilitates generation of alcohol through reductive fermentation by alcohol dehydrogenase from acetaldehyde, the intermediate product, and decreases acetaldehyde content. Since fermentation liquid in reductive fermentation is strongly reductive, proliferation of acetic bacteria is suppressed, and thus, rancidity is never caused.

In reductive maturation of beer (Fig. 2), the jet flow circulation processing apparatus with ultrafine bubbles of hydrogen (Fig. 2) provides DPPH radical-scavenging ability to ingredients of beer, so that beer having an antioxidative function is yielded characteristically.

### Examples

### Example 1: Test of influence of reductive processing in alcohol fermentation

### 1) Method of test

Since there are conditions that should be precisely controlled for a function of generating alcohol by reductive fermentation, such as temperature administration and setting conditions for a control to be compared, small-scale experiments were conducted using a laboratory test apparatus shown in Fig. 1.

The apparatus included a 2-liter beaker and a high-speed magnetic stirrer equipped with a strong magnet; one (1) liter of yeast culture medium to be treated (glucose solution) was placed in the beaker, and an inner lid was disposed blow the surface of the culture medium to prevent involution of air, because when the stirrer rotor is started to rotate, a vortex flow is generated and the stirrer rotor causes air to be engulfed as bubbles in the flow. In the apparatus, a small pipe for feeding hydrogen gas from a supply apparatus of hydrogen gas was disposed so as to extend from the center of the inner lid to just above the stirrer rotor, and thus, minute bubbles of hydrogen gas are generated within the beaker by feeding hydrogen gas while stirring the solution.

That is, for reductive processing, a simple apparatus (Fig. 1) was employed where the stirrer rotor is vigorously rotated at a speed of 3,000 rpm to dissolve hydrogen gas in the culture medium while preventing involution of air by the inner lid.

Though performance of the simple apparatus is far inferior to that of an apparatus of nanobubbles of hydrogen, results of reductive processing have substantially the same tendency as those when an apparatus of nanobubbles of hydrogen was used.

Using the simple reductive processing apparatus, generation of carbon dioxide gas in the stage of alcohol fermentation by yeast and change in oxidation-reduction potential between before and after fermentation were measured.
(1) Yeast was prepared preliminarily in the following manner: yeast was grown in YPD liquid medium, adjusted to be 1.0 × 10⁸ in number of various yeast fungi, washed twice with saline, centrifuged and the supernatant was discarded.
(2) Reduction of glucose solution was performed in the following manner: one (1) liter of 10 % glucose solution was prepared, and, using the simple oxidative-reductive processing apparatus shown in Fig. 1, hydrogen gas was fed at a flow rate of 50 ml per minute while stirring at 3,000 rpm for 10 minutes. As a control, a 10 % glucose solution which had not been processed was prepared.
(3) In preparation of mash, for each of the 10 % glucose solution of the control and the 10 % glucose solution having been subjected to reductive processing, 100 ml was taken out and placed in a 300 ml Erlenmeyer flask, yeast was added thereto, pH, oxidation-reduction potential and weight were measured, and a fermentation tube was attached.

Fermentation was performed in a thermostatic chamber at 25°C for 6 days, during which weight was measured every day to determine loss in weight due to volatilization of carbon dioxide gas. The loss in weight indicates the amount of generated alcohol.

### 2) Results of test

### (1) Course of generation of carbon dioxide gas

Generation of carbon dioxide gas proceeds along the formula

C₆H₁₂O₆ → 2C₂H₅OH + 2CO₂ ↑

and thus, an amount of carbon dioxide gas is an indication of generation of alcohol. Therefore, loss in weight is shown in Table 5.

### (2) Loss in weight due to generation of carbon dioxide gas

**Table 5**

| Course Of Weight Loss Due To Generation Of Carbon Dioxide Gas In Fermentation | | | | | | | |
|---|---|---|---|---|---|---|---|
| Day No. | Day 0 | Day 1 | Day 2 | Day 3 | Day 4 | Day 5 | Day 6 |
| Reduction Processed Section | 0 | 0.8 | 2.0 | 2.9 | - | 4.1 | 4.2 |
| Control Section | 0 | 0.7 | 1.7 | 2.5 | - | 3.9 | 4.1 |

### 3) Summary of results of test

Since the weight of the source substrate for fermentation is 10 g of glucose, expected amounts of alcohol and carbon dioxide gas to be generated are 4.8 g. The difference observed is caused by the reductive processing performed initially and only once.

Weight loss at the last day of test is 4.2 g for the reduction processed section and 4.1 g for the control section, which correspond to 87.5 % and 84.5 % of the expected amount, respectively.

From the course, weight loss in initial raise in the reduction processed section is active, and the weight loss of the reduction processed section is still large at the final day at which an equilibrium is reached, which indicate that the amount of alcohol generated is large.

Thus, it was found that reductive processing accelerates generation of alcohol and also increases the yield.

### Example 2: Influence of processing with ultrafine bubbles on savor ingredients of alcoholic beverage

### 1) Method of test

Influence of gas exchange emerges depending on the relationship between processing with ultrafine bubbles (Fig. 2) and alcohol, so that distilled spirit was used as a representative in the test (Results are also the same for whisky, rice wine, wine and beer).

Processing with ultrafine bubbles of hydrogen provided by vacuum cavitation was performed twice, i.e., for 10 minutes and 30 minutes, on 5 liters of a distilled spirit product to see a trend of savor ingredients by gas chromatography.

### 2) Results of test

Concentration (ppm) Of Savor Ingredients Of Distilled Spirit Sample After Processing With Ultrafine Bubbles (Indices Are Expressed As Relative Values, With Controls Being 100)

| | Processing Time | | | | | |
|---|---|---|---|---|---|---|
| Savor Ingredient | Control (No Processing) | Index Of The Same | Processing For 10 Minutes | Index Of The Same | Processing For 30 Minutes | Index Of The Same |
| Ethyl Acetate | 65 | 100 | 56 | 86 | 45 | 69 |
| Isoamyl Acetate | 11 | 100 | 9 | 81 | 7 | 63 |
| Isobutyl Alcohol | 214 | 100 | 212 | 99 | 200 | 93 |
| Isoamyl Alcohol | 415 | 100 | 408 | 98 | 400 | 96 |
| n-Propyl Alcohol | 140 | 100 | 135 | 96 | 130 | 93 |
| Acetaldehyde | 38 | 100 | 31 | 82 | 26 | 68 |

### 3) Summary of results of test

Decrease of savor ingredients due to processing with ultrafine bubbles is affected greatly by duration of processing time. Volatile ingredients were especially decreased due to gas exchange.

According to the results of measurement by gas chromatography, concentrations of acetaldehyde, ethyl acetate and isoamyl acetate, which are highly volatile, decreased greatly.

Other savor ingredients, namely, isobutyl alcohol, isoamyl alcohol and n-propyl alcohol did not decrease very much.

In particular, acetaldehyde is an ingredient affecting a human body, so that aldehyde is removed, in distilled spirit, based on difference in boiling point between ingredients, however, as for brewed alcoholic beverages, since no distillation step is included while pasteurization is performed, if aldehyde can be removed by gas exchange even at ordinary temperature, high quality alcoholic beverages that do not lead to drunken sickness can be produced.

### Example 3: Production of alcoholic beverage with antioxidative function by processing with ultrafine bubbles

### 1) Method of test

Effect of an antioxidative function is provided to alcohol by processing with ultrafine bubbles, so that distilled spirit was used as a representative in the test (Results are also the same for whisky, rice wine, wine and beer).

Processing with ultrafine bubbles of hydrogen provided by vacuum cavitation was performed twice, i.e., for 10 minutes and 30 minutes, on 5 liters of a distilled spirit product to see a trend of the antioxidative function of the generated alcoholic beverage by measuring scavenging ability thereof against DPPH radicals.

### 2) Results of test

Oxidation-Reduction Potential And DPPH Radical-Scavenging Ability Of Distilled Spirit By Processing With Ultrafine Bubbles

| Processing Time | No Processing | Processing For 10 Minutes | Processing For 30 Minutes |
|---|---|---|---|
| Oxidation-Reduction Potential | +230mV | -700 mV | -750 mV |
| DPPH Radical-Scavenging Rate (%) | 0 | 2.35 | 2.80 |
| Radical-Scavenging Ability (µM/L/min) | 0 | 1.18 | 1.40 |

### 3) Summary of results of test

For the unprocessed section which had not been subjected to reductive processing, the oxidation-reduction potential was +230 mV and the DPPH radical-scavenging ability was 0. For the section subjected to processing with ultrafine bubbles for 10 minutes, the oxidation-reduction potential was -700 mV and the radical-scavenging ability was 1.18 µM/L/min.

For the section subjected to processing with ultrafine bubbles for 30 minutes, the oxidation-reduction potential was -750 mV and the radical-scavenging ability was 1.40 µM/L/min.

Thus, it was confirmed that an antioxidative function is provided by processing with ultrafine bubbles. Therefore, it was found that production of alcoholic beverages having an antioxidative function can be performed by processing with ultrafine bubbles.

### Example 4: Stability of alcoholic beverage having antioxidative function

### 1) Method of test

Processing with ultrafine bubbles of hydrogen provided by vacuum cavitation was performed for 30 minutes on 5 liters of a distilled spirit product, then the generated alcoholic beverage was fractionated, sealed tightly and left stand for one month, two months and three months, to see stability of the antioxidative function. Analysis of the antioxidative function was performed by measuring scavenging ability against DPPH radicals.

### 2) Results of test

Time Course Of Oxidation-Reduction Potential And Radical-Scavenging Ability Of Distilled Alcoholic Spirit Subjected To Processing With Nanobubbles Of Hydrogen

| Elapsed Time | Immediate | After 1 Month | After 2 Months | After 3 Months |
|---|---|---|---|---|
| Oxidation-Reduction Potential (mV) | -750 | -720 | -710 | -720 |
| DPPH Radical-Scavenging Rate (%) | 2.80 | 2.55 | 2.46 | 2.48 |
| Radical-Scavenging Ability (µM/L/min) | 1.40 | 1.28 | 1.23 | 1.24 |

### 3) Summary of results of test

Though there was some experimental error in each processing, the oxidation-reduction potential increased slightly in three months, showing no large change, and the DPPH radical-scavenging ability remained within a range of 1.23 to 1.40, and thus no large change was observed.

Variation in the data is believed to be within experimental error.

That is, it has been found that the antioxidative function acquired by processing with ultrafine bubbles of hydrogen lasts for a long period.

### Industrial applicability

The reductive fermentation technique has a great influence on a series of enzymatic action on generation of alcohol from glucose, so that it accelerates generation of alcohol and increases yield of alcohol, leading to great enhancement in production efficiency as well as decrease of acetaldehyde.

The oxidative proliferation of microorganisms and reductive fermentation brewing method for production of alcoholic beverages decrease factors for occurrence of oxidized odor such as generation of smell of potato, odor of oxidized lipid and browning in the heating process by reductive processing in the stage of processing the base material, compared to the conventional brewing technology where decrease of the factors was left to nature, enabling brewing of alcoholic beverages such as rice wine, wine, distilled spirit, whisky and beer to produce enjoyable refreshing new savor.

Further, utilization of ultrafine bubbles provided by air or oxygen in saccharification, making of malt, production of fermentation starters, and proliferation of yeast creates an oxidative condition, facilitating proliferation of microorganisms and diminishing days for processing before commencement of fermentation greatly, and thus, is important in decreasing production cost.

The processing with ultrafine bubbles not only is involved in generation rate of alcohol and yield thereof but also decreases acetaldehyde in alcoholic beverages, which is a cause of a hangover, through gas exchange, greatly increasing quality of alcoholic beverages.

Moreover, provision of an antioxidative function by processing with ultrafine bubbles is important in that alcoholic beverages are reacknowledged as medicine useful for health promotion, especially in rice wine and wine, which are brewed alcoholic beverages, the antioxidative function is exerted as alcoholic beverages with a small acetaldehyde content, and thus, importance thereof as "an alcoholic beverage is truly the best medicine" is enhanced.

In particular, reductive maturation by processing with ultrafine bubbles for brewed alcoholic beverages and distilled alcoholic beverages provides a strong antioxidative function, so that generation of acetaldehyde in the liver after drinking of an alcoholic beverage is suppressed and a hangover is prevented, and that the antioxidative function has a medicinal benefit of preventing various diseases, therefore application as medicine is broadened.

## Claims

1. A reductive alcoholic beverage containing ultrafine bubbles of hydrogen of a brewed alcoholic beverage and a distilled alcoholic beverage, having an antioxidative function due to a function of ultrafine bubbles of hydrogen to generate reductive radicals.

2. A production method of a reductive alcoholic beverage containing hydrogen with an antioxidative function of a brewed alcoholic beverage and a distilled alcoholic beverage, wherein
a storage tank of an alcoholic beverage, a hydrogen supply apparatus and a processing apparatus with ultrafine bubbles are connected with a communication pipe,
an alcoholic beverage in the storage tank such as a brewed alcoholic beverage and a distilled alcoholic beverage is subjected to processing with ultrafine bubbles of hydrogen gas, and
the alcoholic beverage contains reductive radicals.

3. A low-temperature removing method of acetaldehyde of a brewed alcoholic beverage and a distilled alcoholic beverage, wherein
a storage tank of an alcoholic beverage, a processing apparatus with ultrafine bubbles and a gas supply apparatus, and a communication pipe to circulate the alcoholic beverage are used,
the alcoholic beverage is subjected to processing with ultrafine bubbles of single-component gas of hydrogen, nitrogen, carbon dioxide or the like or of mixed gas of two or more kinds of hydrogen, nitrogen and carbon dioxide, and
contained acetaldehyde, which is a cause of a hangover, is removed by a gas exchanging function of ultrafine bubbles of the various kinds of gas at ordinary temperature or low temperature.

4. A reductive fermentation method of alcohol, wherein
an alcohol fermentation tank, a piston pump, a supply apparatus of nitrogen gas or carbon dioxide gas and a microbubble generation processing apparatus are connected with a communication pipe,
oxygen supply into the alcohol fermentation tank is blocked by continuous circulation processing of microbubbles of the nitrogen gas or the carbon dioxide gas into the fermentation tank,
alcohol dehydrogenase is activated in a direction toward generation of alcohol by creation of a reductive condition by yeast fungi accompanying their own respiration,
generation of alcohol from acetaldehyde which is a intermediate product is facilitated,
remaining acetaldehyde is removed by a gas exchange function of microbubbles of the nitrogen gas or the carbon dioxide gas,
acetaldehyde content is lowered so that a yield of alcohol is increased, and
the nitrogen gas or the carbon dioxide gas is infused forcibly.

5. A reductive fermentation method of alcohol by hydrogen, wherein
an alcohol fermentation tank, a piston pump, a hydrogen supply apparatus and a bubble generation processing apparatus are connected,
microbubbles of hydrogen are circulated to perform forcibly rapid reductive processing in the alcohol fermentation tank, and activity of alcohol dehydrogenase is activated in a direction toward generation of alcohol by continuous circulation reductive processing with microbubbles of hydrogen,
generation of alcohol from acetaldehyde which is an intermediate product is facilitated,
remaining acetaldehyde is removed by a gas exchanging function of minute bubbles of hydrogen gas,
acetaldehyde content is lowered so that a yield of alcohol is increased, and
minute bubbles of hydrogen gas is circulated forcibly into the fermentation tank.

6. A reductive fermentation apparatus composed of an alcohol fermentation tank, a piston pump, a supply apparatus of nitrogen, a supply apparatus of carbon dioxide gas, a supply apparatus of hydrogen gas, a microbubble generation apparatus to generate minute gas bubbles, a condensation-homogenization apparatus of minute gas bubbles, and a pipe to circulate minute gas bubbles,
wherein fermentation converting acetaldehyde to alcohol is facilitated to increase a yield of alcohol, and
a concentration of acetaldehyde is decreased.

7. An oxidative-reductive brewing method comprising
a reductive processing apparatus system of a fermentation base material composed of a water immersion tank of a base material or a juice tank of a base material, a supply apparatus of purged hydrogen gas, and an ultrafine bubble apparatus of hydrogen gas by resonance foaming/vacuum cavitation,
a proliferation apparatus system of healthy yeast composed of a fermentation starter tank, a supply apparatus of purged air or oxygen gas, a piston pump, and a generation apparatus of microbubbles of air,
a reductive fermentation apparatus system composed of an alcohol fermentation tank, a piston pump, a supply apparatus of purged hydrogen gas, a generation apparatus of microbubbles of hydrogen, all the systems being made to operate, and
a producing system of an alcoholic beverage containing reductive radicals composed of a storage tank of an alcoholic beverage, a supply apparatus of purged hydrogen gas, and an ultrafine bubble apparatus of hydrogen gas by resonance foaming/vacuum cavitation,
wherein in water immersion and heating of the base material, water containing ultrafine bubbles of hydrogen gas is used to suppress odor of oxidized lipid, smell of potato, and browning/oxidation of ingredients so as to clear savor,
in proliferation of yeast fungi, ultrafine bubbles of oxygen gas are supplied to facilitate proliferation of healthy yeast so that a growth period thereof is shortened,
in a stage of fermenting mash, initially the fermentation tank and the generation apparatus of microbubbles of air operated by the piston pump are connected and minute gas bubbles of air are supplied to increase a number of yeast fungi,
when the number of yeast fungi is sufficient, switching to alcohol fermentation, minute gas bubbles are supplied by the generation apparatus of microbubbles of hydrogen operated by the piston pump to reduce rapidly an inside of the fermentation tank,
an activity direction of alcohol dehydrogenase is activated toward production of alcohol, and simultaneously inhibit intrusion of pasteurization bacteria during fermentation,
a yield of alcohol is increased and simultaneously acetaldehyde content is decreased, and
when alcohol fermentation ends, an undistilled alcoholic beverage having been wrung out is reduced by the ultrafine bubble apparatus of hydrogen gas to provide an antioxidative function to the undistilled alcoholic beverage, so that the undistilled alcoholic beverage can be stored for a long period as protected against intrusion of acetic bacteria or the like despite being undistilled.

8. An oxidative-reductive brewing apparatus composed of
a reductive processing system of a base material composed of a water immersion tank of a base material or a juice tank of a base material, a supply apparatus of purged hydrogen gas, and an ultrafine bubble apparatus of hydrogen gas by resonance foaming/vacuum cavitation,
a proliferation system of healthy yeast composed of a fermentation starter tank, a supply apparatus of purged air or oxygen gas, a piston pump, and a generation apparatus of microbubbles of air,
a reductive fermentation system composed of an alcohol fermentation tank, a piston pump, a supply apparatus of purged hydrogen gas, and a generation apparatus of microbubbles of hydrogen, and
a producing system of an alcoholic beverage containing reductive radicals, composed of a storage tank of an alcoholic beverage at a stage after an end of fermentation, a supply apparatus of purged hydrogen gas, and an ultrafine bubble apparatus of hydrogen gas,
wherein performing, within a same fermentation tank, facilitation of proliferation of microorganisms under an oxidative condition and a reductive fermentation technique is enabled in brewing various kinds of alcoholic beverages.
